# EUROPÄISCHE PATENTSCHRIFT

(11) **EP 1 890 672 B1**
(45) Veröffentlichungstag und Bekanntmachung des Hinweises auf die Patenterteilung: **02.06.2010**
(21) Anmeldenummer: 06724793.2
(22) Anmeldetag: 11.05.2006
(51) Int. Cl.: A61K 8/49, A61Q 19/02, A61Q 19/08, A61P 17/00, A61K 31/5377, A61K 31/496, A61K 31/4433, C07D 405/06, C07D 413/06

(54) **VERWENDUNG VON CHROMONDERIVATEN**
USE OF CHROMONE DERIVATIVES
UTILISATION DE DERIVES DE CHROMONE

(30) Priorität: 02.06.2005 DE 102005025801
(43) Veröffentlichungstag der Anmeldung: 27.02.2008
(73) Patentinhaber: Merck Patent GmbH, 64293 Darmstadt (DE)
(72) Erfinder: MUJICA-FERNAUD, Teresa, 64289 Darmstadt (DE); CAROLA, Christophe, 69120 Heidelberg (DE); BUCHHOLZ, Herwig, 60599 Frankfurt (DE)
(86) Internationale Anmeldenummer: PCT/EP2006/004427
(87) Internationale Veröffentlichungsnummer: WO 2006/128562

(56) Entgegenhaltungen:
- EP-A- 0 580 503
- EP-A- 1 508 327
- ROMUSSI, G. ET AL: "Mannich bases from methoxychromones" FARMACO, EDIZIONE SCIENTIFICA , 32(9), 635-9 CODEN: FRPSAX; ISSN: 0430-0920, 1977, XP009069618
- DATABASE CA [Online] CHEMICAL ABSTRACTS SERVICE, COLUMBUS, OHIO, US; CHEN, YUANWEI ET AL: "Preparation of substituted 3,5-dihydro-4H-imidazol-4-ones for the treatment of obesity" XP002391803 gefunden im STN Database accession no. 2004:565223 -& WO 2004/058727 A1 (BAYER PHARMACEUTICALS CORPORATION, USA) 15. Juli 2004 (2004-07-15)
- PATENT ABSTRACTS OF JAPAN Bd. 018, Nr. 107 (C-1169), 22. Februar 1994 (1994-02-22) -& JP 05 301813 A (KAO CORP), 16. November 1993 (1993-11-16) in der Anmeldung erwähnt

## Beschreibung

Gegenstand der Erfindung ist die nicht-therapeutische Verwendung von Chromonderivaten zur Prophylaxe gegen Pigmentstörungen wie Hyperpigmentierung (Chloasma), Sommersprossen (Epheliden), Altersflecken (Lentigines), Sonnenflecken (Lentigo solaris) sowie umweltbedingter Hautalterung.
Ferner betrifft die Erfindung Zubereitungen mit einem wirksamen Gehalt an Chromon-Derivaten. Insbesondere betrifft die vorliegende Erfindung kosmetische Zubereitungen zur Prophylaxe und/oder Behandlung von Pigmentstörungen wie Hyperpigmentierung, Sommersprossen, Altersflecken, Sonnenflecken sowie auch umweltbedingter Hautalterung.

90% aller 50jährigen Frauen leiden an Pigmentflecken, aber die unschönen Tupfer, die sich vorwiegend an Dekolleté und Händen zeigen, sind auch bei 40jährigen keine Seltenheit. Altersflecken entwickeln sich aber auch bei Frauen, die regelmäßig die Pille einnehmen, während der Schwangerschaft oder durch UV-Licht. Sonnen- und Altersflecken treten bei Frauen häufiger auf wie bei Männern. Altersflecken sind völlig harmlos und zeigen auch nach Jahren keine Tendenz zur bösartigen Umwandlung.

Haut- und Haarfarbe sind abhängig vom Gehalt, Größe und Typ des Melanins (einem stickstoffhaltigen dunklen Farbstoff) welches aus Melanozyten, den zur Melaninbildung befähigten Zellen, produziert wird. Ausgehend von Tyrosin und der Hilfe von verschiedenen Melanozyten spezifischen Enzymen wie Tyrosinase oder Tyrosinase-verwandten Proteinen, wird Melanin innerhalb der Melanosomen produziert, mit anschließender Umwandlung der Melanosomen in Keratinozyten. Obwohl das Melanin in der Haut ein geeigneter Schutz gegen UV-Strahlung ist, kann dunklere oder überpigmentierte Haut, wie schon erwähnt, die Schönheit beeinflussen und zu ernsthaften ästhetischen Problemen führen. Hyperpigmentierte Hautbedingungen oder Läsionen enthalten Melasma (auch Chloasma genannt), d.h. unregelmäßig gestaltete gelblich-braune Flecken.
Generell unterscheidet man bei Pigmentflecken zwischen Sommersprossen (Epheliden), Altersflecken (Lentigines), sogenannten Alterswarzen (Verrucae seborrhoicea) und einer Hyperpigmentierung (z.B. Chloasma oder Melasma) und sehr häufig spielt die Sonne hier eine wichtige Rolle. Zu Sommersprossen neigen vor allem Menschen mit sehr heller Haut und rötlichen Haaren. Hyperpigmentierung (Chloasma) findet man hingegen häufig bei jenen Frauen, die regelmäßig ihrem Körper Östrogene zuführen. Vorbeugen kann man vor allem durch regelmäßigen Sonnenschutz mit einem hohen Lichtschutzfaktor. Ist es aber einmal passiert, so bieten sich verschiedene Möglichkeiten wie Laser, Dermabrasio oder andere elektrochirurgische Verfahren sowie sogenannte Bleichcremes an, um die unschönen Altersflecken zu entfernen. Letztere Alternative (Bleichcremes) hat den Vorteil, dass sie für den Patienten wesentlich kostengünstiger als die elektrochirurgischen Verfahren ist.

Daher war es Aufgabe der vorliegenden Erfindung neue Verbindungen herzustellen, die die Fähigkeit zur Hautaufhellung besitzen.
Eine große Zahl von Verbindungen mit hautaufhellender Wirkung zur Behandlung von Pigmentflecken ist auf dem Markt verfügbar.
Unter anderem sind dies Verbindungen wie Hydrochinon (ist als Wirkstoff in der Kosmetik nicht mehr erlaubt), Kojicsäure, Arbutin, Aloesin oder Rucinol, die die Melaminproduktion in der Haut unterbinden. Sie verzögern die Umwandlung von Tyrosin in Melanin durch Blockade des Enzyms Tyrosinase.

Diese Verbindungen haben jedoch eine Reihe von Nachteilen, wie z.B. geringe Depigmentierungs-Effizienz, Nebenwirkungen wie Hautirritationen oder Hautexfoliation (Hautabschälung), Zellschädigungen, geringe Hautdurchdringung oder geringe Haltbarkeit der Formulierungen. Daher ist ein Bedürfnis nach neuen Hautaufhellern mit höherer Effektivität vorhanden.

Überraschenderweise ist nun gefunden worden, dass bestimmte Chromonderivate der Formel I exzellente Hautaufheller-Eigenschaften besitzen. Sie unterbinden die Synthese von Melanin, verhindern die Melanin-Überproduktion und sind somit zur Behandlung von Pigmentflecken aller Art geeignet.

Gegenstand der vorliegenden Erfindung ist somit die Verwendung gemäß Anspruch 1 von Verbindungen der Formel I oder deren physiologisch unbedenklichen Salzen 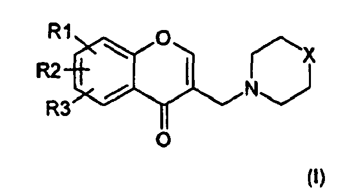 oder einer Zubereitung enthaltend mindestens eine Verbindung der Formel I,
wobei
R₁, R₂ und R₃ -H, -OH, -OA, -A, -OCOA
R₁ und R₂ zusammen können -methylendioxy oder -ethylendioxy sein,
R₁, R₂ und R₃ sind identisch oder unterschiedlich mit der Bedingung, dass sie nicht alle gemeinsam -H sein können,
X -O, -CH -R₄, -N-R₄
R₄ -H, -A, -COOH, -COOA, -COA, -CONH₂, -CONHA
A Alkyl Benzyl, Phenyl

Ein weiterer Gegenstand der vorliegenden Erfindung sind dabei die neuen Chromonderivate der Formeln Ia bis Im

Für die erfindungsgemäße Verwendung werden vorzugsweise Verbindungen der Formel I eingesetzt, bei denen R₁ und R₂ für H und/oder OH stehen.
Insbesondere bevorzugt kann in einer Efindungsvariante ein Chromon-Derivat sein, gekennzeichnet dadurch, dass R₃ für H oder OH steht.
In einer weiteren Erfindungsvariante kann es bevorzugt sein, wenn es sich bei dem Chromon-Derivat nach Formel I um eine Verbindung ausgewählt aus den Verbindungen mit den Formeln 1a bis Im handelt.

Grundsätzlich sind im Sinne der vorliegenden Erfindung von der Bezeichnung "Verbindung nach Formel I" auch die Salze der Verbindungen nach Formel I umfasst. Zu den bevorzugten Salzen gehören dabei insbesondere Alkali- und Erdalkalimetallsalze sowie Ammonium-Salze, insbesondere jedoch Natrium- und Kalium-Salze.

Ein weiterer Gegenstand der vorliegenden Erfindung sind Zubereitungen enthaltend mindestens eine Verbindung nach Formel I gemäß Anspruch 1 sowie mindestens einen weiteren hautpflegenden Inhaltsstoff und mindestens einen für topische Anwendungen geeigneten Träger.

Die erfindungsgemäße Verwendung von Verbindungen der Formel I nach Anspruch 1 oder einer Zubereitung enthaltend eine Verbindung der Formel I nach Anspruch 1 ist die nicht therapeutische Verwendung zur Prophylaxe und/oder Behandlung von Pigmentstörungen wie Hyperpigmentierung, Sommersprossen, Altersflecken sowie umweltbedingter Hautalterung.

Noch bevorzugter ist die Verwendung von Verbindungen nach Formel I, wobei R₁ und R₂ für H und/oder OH stehen, da das Wirkpotential von Vertretern dieser Erfindungsklasse im vorher genannten Sinne besonders hoch ist. Weiterhin bevorzugt ist die Verwendung von Verbindungen nach Formel I, die dadurch gekennzeichnet sind, dass R₃ für H oder OH steht.

Insbesondere ist die Verwendung von Chromon-Derivaten ausgewählt aus den Verbindungen mit den Formeln Ia bis Im bevorzugt:

| | |
|---|---|
| 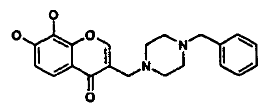 | Ia |
| 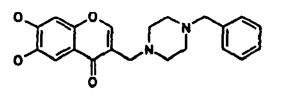 | Ib |
| 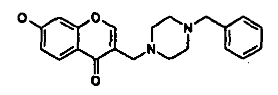 | Ic |
| 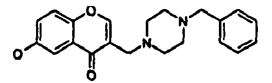 | Id |
| 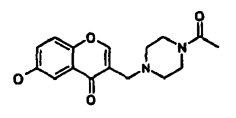 | Ie |
| 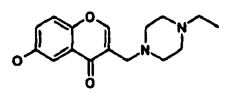 | If |
| 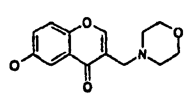 | Ig |
| 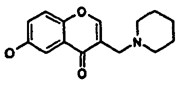 | Ih |
| 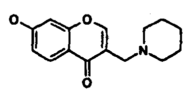 | Ii |
| 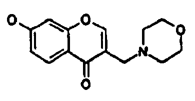 | Ij |
| 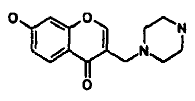 | Ik |
| 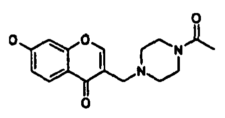 | Im |

Ähnliche Anwendungen strukturell verwandter Verbindungen sind aus der Literatur bekannt:
Aus EP 1216 692 ist die Verwendung von 2-Methyl-2-(β-Carboxyethyl)-Chroman-Derivaten in kosmetischen Zubereitungen bekannt. Die genannten Verbindungen eignen sich besonders zur Prophylaxe gegen Alterungsprozesse von Haut und Haaren sowie zur Prophylaxe gegen trockene Haut, Faltenbildung und Pigmentstörungen.

Zubereitungen zur topischen Anwendung, die Chromon-Derivate, wie z.B. Chromon, 7-Hydroxychromon, 7-Methoxychromon, 5,7-Dihydroxy-2-methyl-chromon, 3-Methyl -2-butenyloxy-chromon, 3-Acetyl-5,7-dihydroxy-2-methyl-chromon, 5-Hydroxychromon, n-Pentyl-7-methoxychromon-2-carboxylat, n-Undecyl-5-methoxychromon-2-carboxylat, 5-Hydroxy-7-methoxy-2-methyl-chromon, 7-Methoxy-chromon-2-carbonsäure, n-Pentyl-chromon-2-carbonsäure, 5-Methoxychromon und Chromon-2-carbonsäure, enthalten, sind aus der japanischen Patentanmeldung JP 05/301813 bekannt. Die Chromon-Derivate wirken als hautverträgliche Tyrosinase-Inhibitoren, welche die Hyperpigmentierung der Haut verringern.

Aus der Japanischen Patentanmeldung JP 09/188608 ist die Verwendung von substituierten Chromon-Derivaten, wie insbesondere 5,7-Dihydroxychromone, 7-Methoxychromone, 5-Hydroxy-7-methoxy-2-methyl-chromon und 5-Hydroxy-2-methyl-chromon, als Wirkstoff gegen graue Haare bekannt. Die Wirkung wird dabei auf die Aktivierung der Farb-pigmentbildenden Zellen und die Steigerung der Melanogenese zurückgeführt.

Aus JP 10/194919 sind Chromon-Derivate bekannt, die in Position 2 mit C₁₋₁₅-Alkyl substituiert sind und in Position 7 H-, OH- oder Alkoxy-Substitution aufweisen. Sie finden Verwendung als Wirkstoffe gegen Hautalterung.

Aus EP 580 503 sind Aminoalkylchromone bekannt, die als Wirkstoff in pharmazeutischen Zubereitungen zur Behandlung von Angst, Depressionen, Psychosen und der Schizophrenie Anwendung finden.

Die Verbindungen der Formel I werden erfindungsgemäß üblicherweise in Mengen von 0.01 bis 20 Gew.%, vorzugsweise in Mengen von 0.1 bis 10 Gew.% eingesetzt. Dabei bereitet es dem Fachmann keinerlei Schwierigkeiten die Mengen abhängig von der beabsichtigten Wirkung der Zubereitung entsprechend auszuwählen.

Die schützende Wirkung gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet, geeignet schnell oder zeitverzögert wirkende Antioxidantien auszuwählen.

In einer bevorzugten Ausführungsform der vorliegenden Erfindung handelt es sich bei mindestens einem weiteren hautpflegenden Inhaltsstoff um ein oder mehrere Antioxidantien und/oder Vitamine.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl- , Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin C und dessen Derivaten, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel V 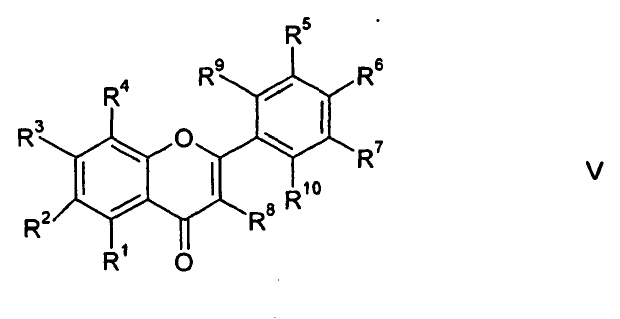 wobei R¹ bis R¹⁰ gleich oder verschieden sein können und ausgewählt sind aus
- H
- OR"
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
- wobei alle OR" unabhängig voneinander stehen für
   - OH
   - geradkettige oder verzweigte C₁- bis C₂₀-Alkyloxygruppen,
   - geradkettigen oder verzweigten C₃- bis C₂₀-Alkenyloxygruppen,
   - geradkettigen oder verzweigten C₁- bis C₂₀-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
   - C₃- bis C₁₀-Cycloalkyloxygruppen und/oder C₃- bis C₁₂-Cycloalkenyloxygruppen, wobei die Ringe jeweils auch durch -(CH₂)ₙ-Gruppen mit n = 1 bis 3 überbrückt sein können und/oder,
   - Mono- und/oder Oligoglycosylreste,
   mit der Maßgabe, dass mindestens 4 Reste aus R¹ bis R⁷ stehen für OH und dass im Molekül mindestens 2 Paare benachbarter Gruppen -OH vorliegen,
- oder R², R⁵ und R⁶ für OH und die Reste R¹, R³, R⁴ und R⁷⁻¹⁰ für H stehen,
wie sie in der älteren Deutschen Patentanmeldung DE 10244282.7 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel V, welches sich im DPPH-Assay in einer hohen Kapazität Radikale zu fangen (EC₅₀), einer zeitverzögerten Wirkung (T_{EC50} > 120 min) und damit einer mittleren bis hohen antiradikalischen Effizienz (AE) äußert. Zudem vereinigen die Verbindungen nach Formel V im Molekül antioxidative Eigenschaften mit UV-Absorption im UV-A- und/oder -B-Bereich. Bevorzugt sind daher auch Zubereitungen enthaltend zumindest eine Verbindung der Formel V, die dadurch gekennzeichnet ist, dass mindestens zwei benachbarte Reste der Reste R¹ bis R⁴ stehen für OH und mindestens zwei benachbarte Reste der Reste R⁵ bis R⁷ stehen für OH. Insbesondere bevorzugte Zubereitungen enthalten zumindest eine Verbindung der Formel V, die dadurch gekennzeichnet ist, dass mindestens drei benachbarte Reste der Reste R¹ bis R⁴ stehen für OH, wobei vorzugsweise die Reste R¹ bis R³ für OH stehen.

Damit die Verbindungen der Formel I ihre positive Wirkung auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Insbesondere eignen sich die erfindungsgemäßen Zusammensetzungen auch zur Behandlung von Hautkrankheiten, die durch UV-Strahlung hervorgerufen werden.

Erfindungsgemäß bevorzugte Zubereitungen enthalten neben den Verbindungen der Formel I auch UV-Filter.

Bei Einsatz der als UV-A-Filter insbesondere bevorzugten Dibenzoylmethanderivate in Kombination mit den Verbindungen der Formel I ergibt sich ein zusätzlicher Vorteil: Die UV-empfindlichen Dibenzoylmethanderivate werden durch die Anwesenheit der Verbindungen der Formel I zusätzlich stabilisiert. Ein weiterer Gegenstand der vorliegenden Erfindung ist daher die Verwendung der Verbindungen gemäß Formel I zur Stabilisierung von Dibenzoylmethanderivaten in Zubereitungen.

Prinzipiell kommen alle UV-Filter für eine Kombination mit den erfindungsgemäßen Verbindungen der Formel I in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-{(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),

Weitere geeignete UV-Filter sind auch Methoxyflavone ensprechend der älteren Deutschen Patentanmeldung DE 10232595.2.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filtemden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxybenzophenon, Methoxyzimtsäureoctylester, 3,3,5-Trimethyl-cyclo-hexyl-salicylat, 4-(Dimethylamino)benzoesäure-2-ethyl-hexylester, 2-Cyano-3,3-diphenyl-acrylsäure-2-ethylhexylester, 2-Phenyl-benzimidazol-5-sulfon-säure sowie ihre Kalium-, Natrium- und Triethanol-aminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, welche die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben. Geeignete Kapseln können Wände aus anorganischen oder organischen Polymeren aufweisen. Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Alle Verbindungen oder Komponenten, die in den Zubereitungen verwendet werden können, sind entweder bekannt und käuflich erwerbbar oder können nach bekannten Verfahren synthetisiert werden. Die Herstellung der neuen Verbindungen nach Formel I wird weiter unten beschrieben.

Eine oder mehrere Chromon-Derivate der Formel können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Öle, Aerosole, Sprays oder auch Sticks. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen.. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z.B. in Kollagenmatrices und anderen üblichen Verkapselungsmaterialien.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben mit einem kosmetisch oder dermatologisch geeigneten Träger vermischt wird, und die Verwendung einer Verbindung der Formel I zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I in dem Träger zur Folge haben.

In einem erfindungsgemäß bevorzugten Verfahren wird die Verbindung gemäß Formel I hergestellt durch die Reaktion eines entsprechend substituierten 2-Hydroxyacetophenones der Formel II (siehe Schema 1) mit Dimethylformamid (DMF) in Gegenwart von POCl₃ zum Aldehyd der Formel III (Vilsmeyer-Haack-Reaktion; siehe analog Tetrahedron Lett. 1996, 52,10, 3669) und anschließender reduktiver Aminierung des Aldehyds mittels eines Amins der Formel IV. Dabei kann die reduktive Aminierung analog zu Abdel-Magid et al., J. Org. Chem. 1996, 61, 2849-3862 erfolgen.

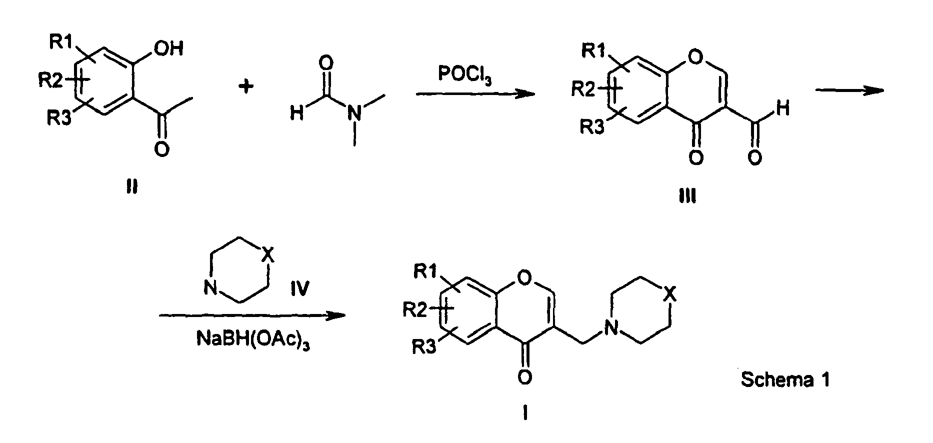

Durch ihre Wirkung eignen sich Verbindungen der Formel I auch als Arzneimittelwirkstoff.

Die folgenden Beispiele sollen die vorliegende Erfindung näher erläutern ohne sie einzuschränken.

### Beispiel 1:

### Herstellung von 3-(4-Benzyl-piperazin-1-ylmethyl)-7,8-dihydroxy-chromen-4-on (Ia)

### - Herstellung der Vorstufe 3-Carbaldehyd-7,8-dihydroxy-chromen-4-on (IIIa)

Zu einer Lösung von 59 mmol (10.0 g) 2,4,6-Trihydroxyacetophenon (IIa) in DMF (100 mL) werden bei -50°C unter Schutzgas 238 mmol (21.8 ml) Phosphorylchlorid zugegeben. Die Reaktionslösung wird noch 30 Min gerührt, dann noch 24 h bei Raumtemperatur und schließlich in 200g Eis gegossen. Das entstehende orange Präzipitat wird abfiltriert, mit Wasser gewaschen und getrocknet. Es entsteht 5.9 g 3-Carbaldehyd-7.8-dihydroxy-chromen-4on (IIIa) als oranger Feststoff.

### - Herstellung von 3-(4-Benzyl-piperazin-1-ylmethyl)-7,8-dihydroxy-chromen-4-on (Ia)

Zu 0.97 mmol (200mg) der Verbindung IIIa und 0.97 mmol (0.17 ml) N-Benzylpiperazin (IVa) in 1,2-Dichlormethan (10ml) werden bei Raumtemperatur unter Schutzgas 1.45 mmol (308 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt und über Wasser (25 ml) und Dichlormethan (25 ml) aufgeteilt. Die wässrige Phase wird zweimal mit jeweils 25 ml Dichlormethan extrahiert und anschließend die vereinigte organische Phase über MgSO₄ getrocknet und das Lösungsmittel im Vakuum abgezogen. Der Feststoff wird vorsichtig aus Ethanol umkristallisiert, sodass die Verbindung la entsteht. ESI-MS: 367 (M+H)⁺

### Beispiel 2:

### - Hemmung des Enzyms Tyrosinase

Die Wirkung der Verbindungen I als Hautaufheller wird geprüft durch Ihre Fähigkeit das Enzym Tyrosinase zu hemmen und damit die Melanin-Synthese zu unterbinden.
Um die hemmende Wirkung der Verbindungen I gegenüber Tyrosinase zu zeigen wurden als Substrate Pilz-tyrosinase und L-Tyrosin oder L-DOPA verwendet und mit der Referenz-Verbindung verglichen.

### - Tyrosinase-Versuch

Die Verbindungen I und Tyrosinase (10 U) werden 10 Min. in Eis vorgekühlt und dann L-DOPA (4 mM) zugefügt und für 1 h auf 37°C gehalten. Es wird die optische Dichte jedes experimentell ermittelten Punktes bei 450 nm gegenüber der entsprechenden Referenz ohne Enzym gemessen. Kojicsäure wird als Tyrosinase-Referenz-Inhibitor benutzt. Die Versuchsergebnisse sind in der folgenden Tabelle dargestellt, ausgedrückt durch den IC 50-Wert (d.h. die Konzentration der jeweiligen Versuchssubstanz bei der die Tyrosinase-Aktivität um 50% blockiert ist).

| Verbindung | IC₅₀ (µM) |
|---|---|
| Kojicsäure | 10 |
| Ia | 57 |
| Ib | 83 |

### Beispiel 3:

### Herstellung von 3-(4-Benzyl-piperazin-1-ylmethyl)-6,7-dihydroxy-chromen-4-on (Ib)

### - Herstellung der Vorstufe 3-Carbaldehyd-6,7-dihydroxy-chromen-4-on (IIIb)

Zu einer Lösung von 5,9 mmol (1.0 g) 2,4,5-Trihydroxyacetophenon (IIa) in DMF (10mL) werden bei -50°C unter Schutzgas 23.8 mmol (2.2 ml) Phosphorylchlorid zugegeben. Die Reaktionslösung wird noch 30 Min gerührt, dann noch 24 h bei Raumtemperatur und schließlich in 40g Eis gegossen. Das entstehende braune Präzipitat wird abfiltriert, mit Wasser gewaschen und getrocknet. Es entsteht 1.02 g 3-Carbaldehyd-6,7-dihydroxy-chromen-4on (IIIb) als brauner Feststoff.

### - Herstellung von 3-(4-Benzyl-piperazin-1-ylmethyl)-7,8-dihydroxy-chromen-4-on (Ib)

Zu 0.97 mmol (200mg) der Verbindung IIIb und 0.97 mmol (0.17 ml) N-Benzylpiperazin (IVa) in 1,2-Dichlormethan (10ml) werden bei Raumtemperatur unter Schutzgas 1.45 mmol (308 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt und über Wasser (25 ml) und Dichlormethan (25 ml) aufgeteilt. Die wässrige Phase wird zweimal mit jeweils 25 ml Dichlormethan extrahiert und anschließend die vereinigte organische Phase über MgSO₄ getrocknet und das Lösungsmittel im Vakuum abgezogen. Der Feststoff wird vorsichtig aus Ethanol umkristallisiert, sodass die Verbindung Ib entsteht.
EI-MS: 366 (M⁺), 324, 220, 191, 91

### Beispiel 4:

### Herstellung von 3-(4-Benzyl-piperazin-1-ylmethyl)-7-hydroxy-chromen-4-on (Ic)

### - Herstellung der Vorstufe 3-Carbaldehyd-7-hydroxy-chromen-4-on (IIIc)

Zu einer Lösung von 131.5 mmol (20.0 g) 2,4-Dihydroxyacetophenon (IIc) in DMF (240 mL) werden bei -70°C unter Schutzgas 512.7 mmol (47.9 mL) Phosphorylchlorid zugegeben. Die Reaktionslösung wird noch 30 Min gerührt, dann noch 24 h bei Raumtemperatur und schließlich 2 Stunden auf 50° geheizt. Dann wurde das Reaktiongemisch auf Raumtemperatur heruntergekühlt und schließlich in 400g Eis gegossen. Das entstehende orange/rot Präzipitat wird abfiltriert, mit Wasser gewaschen und getrocknet. Es entsteht 17.0 g 3-Carbaldehyd-7-hydroxy-chromen-4on (IIIc) als roter Feststoff. Diese Substanze wurde in 300 mL Dichloromethane/Methanol lösung (9/2) gelöst, über Kieselgel gegeben und mit 2L Eluent nachgewashen und das Lösemittel einrotiert.
Ausbeute: 11.8 g

### - Herstellung von 3-(4-Benzyl-piperazin-1-ylmethyl)-7-hydroxy-chromen-4-on (Ic)

Zu 1.05 mmol (200mg) der Verbindung IIIc und 1.05 mmol (0.18 ml) N-Benzylpiperazin (IVa) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt und über Wasser (25 ml) und Dichlormethan (25 ml) aufgeteilt. Die wässrige Phase wird auf pH= 8 eingestellt, zweimal mit jeweils 25 ml Dichlormethan extrahiert und anschließend die vereinigte organische Phase über MgSO₄ getrocknet und das Lösungsmittel im Vakuum abgezogen. Es entsteht 150 mg 3-(4-Benzyl-piperazin-1-ylmethyl)-7-hydroxy-chromen- 4-on Ic
EI-MS: 350 (M⁺), 308, 204, 175, 91
1H NMR (300 MHz, DMSO-d6) δ 8.08 (s, 1H), 7.88 (d, *J*= 8.8 Hz, 1H), 7.34-7.20 (m, 5H), 6.90 (dd, *J*= 8.7, 2.3 Hz, 1H), 6.81 (d, *J* = 2.2 Hz, 1H), 3.44 (s, 2H), 3.30 (s, 2H), 2.5-2.3 (m, 8H).

### Beispiel 5:

### Herstellung von 3-(4-Benzyl-piperazin-1-ylmethyl)-6-hydroxy-chromen-4-on (Id)

### - Herstellung der Vorstufe 3-Carbaldehyd-6-hydroxy-chromen-4-on (IIId)

Zu einer Lösung von 6.57 mmol (1.0 g) 2,5-Dihydroxyacetophenon (IId) in DMF (20 mL) werden bei -50°C unter Schutzgas 26.28 mmol (2.4 mL) Phosphorylchlorid zugegeben. Die Reaktionslösung wird noch 2 Stunden gerührt, dann noch 24 h bei Raumtemperatur und schließlich wurde das Reaktionsgemisch in 50 mL Eis gegossen. Das entstehende gelbe Präzipitat wird abfiltriert, mit Wasser gewaschen und getrocknet. Es entsteht 1.14 g 3-Carbaldehyd-6-hydroxy-chromen-4on (IIId) als gelber Feststoff.

### - Herstellung von 3-(4-Benzyl-piperazin-1-ylmethyl)-6-hydroxy-chromen-4-on (Id)

Zu 1.05 mmol (200mg) der Verbindung IIId und 1.16 mmol (0.20 ml) N-Benzylpiperazin (IVa) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Der Feststoff wird vorsichtig aus Dichloromethan umkristallisiert, sodass die Verbindung Id entsteht (260 mg).
EI-MS: 350 (M⁺), 308, 204, 175, 91
1H NMR (300 MHz, DMSO-d6) δ 9.91 (bs, 1H), 8.15 (s, 1H), 7.48 (d, *J*= 9.1 Hz, 1H), 7.34 (d, *J*= 2.8 Hz, 1H), 7.32-7.24 (m, 5H), 7.21 (dd, *J*= 9.1, 2.8 Hz, 1H), 3.44 (s, 2H), 3.33 (s, 2H), 2.47-2.40 (m, 4H), 2.40-2.32 (m, 4H).

### Beispiel 6:

### Herstellung von 3-(4-Acetyl-piperazin-1-ylmethyl)-6-hydroxy-chromen-4-on (Ie)

Zu 1.05 mmol (200mg) der Verbindung IIId und 1.16 mmol (0.15 ml) N-Acetyllpiperazin (IVb) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Der Feststoff wird vorsichtig aus Dichloromethan umkristallisiert, sodass die Verbindung Id entsteht als beiger Feststoff (260 mg).
EI-MS: 302 (M⁺), 230, 218, 176, 125, 85
1H NMR (300 MHz, DMSO-d6) δ 10.01 (s, 1H), 8.25 (s, 1H), 7.52 (d, *J*= 8.9 Hz, 1H), 7.35 (d, *J*=2.8, 1H), 7.23(dd, *J*= 8.9, 2.8 Hz, 1H), 3.40 (m, 5H)), 3.30 (m, 5H), 1.98 (s, 3H).

### Beispiel 7:

### Herstellung von 3-(4-Ethyl-piperazin-1-ylmethyl)-6-hydroxy-chromen-4-on (If)

Zu 1.05 mmol (200mg) der Verbindung IIId und 1.16 mmol (0.15 ml) N-Ethylpiperazin (IVc) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Es entsteht 343 mg 3-(4-ethyl-piperazin-1-ylmethyl)-6-hydroxy-chromen-4-on (If) als oranger Feststoff.
APCI-MS: 289 (M+H)⁺

### Beispiel 8:

### Herstellung von 6-Hydroxy-3-morpholin-4-ylmethyl-chromen-4-on (Ig)

Zu 1.05 mmol (200mg) der Verbindung IIId und 1.10 mmol (0.15 ml) Morpholin (IVd) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Es entsteht 334 mg 3-(Morpholin-1-ylmethyl)-6-hydroxy-chromen-4-on (Ig) als oranger Feststoff.
EI-MS: 261 (M⁺), 203, 176, 86
1H NMR (300 MHz, DMSO-d6) δ 8.26 (s, 1H), 7.54 (d, *J*= 9.0 Hz, 1H), 7.38 (d, *J* = 2.8 Hz, 1H), 7.27 (dd, *J*= 9.0, 2.8 Hz, 1H), 3.60 (m, 4H), 3.37 (s, 2H), 2.45 (m, 4H).

### Beispiel 9:

### Herstellung von 6-Hydroxy-3-piperidin-1-ylmethyl-chromen-4-on (Ih)

Zu 1.05 mmol (200mg) der Verbindung IIId und 1.16 mmol (0.12 ml) Piperidin (IVe) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase mit jeweils 25 ml Ethylacetat extrahiert und anschließend die vereinigte organische Phase über MgSO₄ getrocknet und das Lösungsmittel im Vakuum abgezogen. Es entsteht 216 mg 3-(Piridin-1-ylmethyl)-6-hydroxy-chromen-4-on (Ih) als orange Feststoff.
EI-MS: 259 (M⁺), 216, 176, 84
1H NMR (300 MHz, DMSO-d6) δ 8.22 (s, 1H), 7.55 (d, *J*= 8.9 Hz, 1H), 7.39 (d, *J* = 2.8 Hz, 1H), 7.27 (dd, *J*= 8.9, 2.8 Hz, 1H), 3.34 (s, 2H), 2.42 (m, 4H), 160-1.34 (m, 6H)

### Beispiel 10:

### Herstellung von 7-Hydroxy-3-piperidin-1-ylmethyl-chromen-4-on (Ii)

Zu 1.05 mmol (200mg) der Verbindung IIIc und 1.05 mmol (0.11 ml) Piperidin (IVe) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird mit jeweils 25 ml Ethylacetat extrahiert und anschließend die vereinigte organische Phase über MgSO₄ getrocknet und das Lösungsmittel im Vakuum abgezogen. Der Feststoff wird vorsichtig aus MTBE umkristallisiert, sodass die Verbindung Ih entsteht als leicht-oranger Feststoff (157 mg).
EI-MS: 259 (M⁺), 216, 176, 84
1H NMR (300 MHz, DMSO-d6) δ 8.22 (s, 1H), 7.55 (d, *J*= 8.9 Hz, 1H), 7.39 (d, *J* = 2.8 Hz, 1H), 7.27 (dd, *J*= 8.9, 2.8 Hz, 1H), 3.34 (s, 2H), 2.42 (m, 4H), 160-1.34 (m, 6H)

### Beispiel 11:

### Herstellung von 7- Hydroxy-3-morpholin-4-ylmethyl-chromen-4-on (Ij)

Zu 1.05 mmol (200mg) der Verbindung IIIc und 1.05 mmol (0.10 ml) Morpholin (IVd) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Es entsteht 3-(Morpholin-1-ylmethyl)-7-hydroxy-chromen-4-on (Ij) als rosa Feststoff (230 mg).
EI-MS: 261 (M⁺), 243, 176, 86
1H NMR (300 MHz, DMSO-d6) δ 8.32 (s, 1H), 8.07 (d, *J*= 8.7 Hz, 1H), 7.10 (dd, *J*= 8.7, 2.3 Hz, 1H), 7.02 (d, *J* = 2.3 Hz, 1H), 3.75 (m, 2H), 3.45 (m, 6H), 2.60 (m, 2H).

### Beispiel 12:

### Herstellung von 7-Hydroxy-3-piperazin-1-ylmethyl-chromen-4-on (Ik)

Zu 1.05 mmol (200mg) der Verbindung IIIc und 1.05 mmol (196 mg) N-Boc-Piperazin (IVe) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Das Rohprodukt wird durch Säulenchromatographie aufgereinigt CH₂Cl₂: MeOH, (98:2).
250 mg der 7- Hydroxy-3-(4-Boc-piperazin-1-ylmethyl-chromen-4-on Derivative (0.69 mmol) werden bei Raumtemperatur unter Schutzgas in 8 mL CH₂Cl₂ gelöst. 1.2 mL Trifluoressigsäure (15.58 mmol) zugegeben und das Reaktionsgemisch 24 h gerührt. Danach wird das Lösungsmittel im Vakuum abgezogen. Der Feststoff wird aus EtOH umkristallisiert, sodass die Verbindung Ik als leicht-rosa Feststoff (120 mg) entsteht.
EI-MS: 260 (M⁺), 218, 175, 85

### Beispiel 13:

### Herstellung von 3-(4-Acetyl-piperazin-1-ylmethyl)-7-hydroxy-chromen-4-on (Im)

Zu 1.05 mmol (200mg) der Verbindung IIIc und 1.05 mmol (135 mg) N-Acetylpiperazin (IVb) in 1,2-Dichlormethan (10ml) werden bei Raumtemperatur unter Schutzgas 1.47 mmol (312 mg) Natrium-triacetoxyborhydrid sowie 1 Tr. Essigsäure zugegeben und das Reaktionsgemisch 24 h gerührt. Danach wird das Reaktionsgemisch mit kalter wässriger Lösung abgeschreckt. Die wässrige Phase wird auf pH= 8-9 eingestellt, zweimal mit jeweils 25 ml Ethylacetat extrahiert und anschließend die vereinigte organische Phase über MgSO₄ getrocknet und das Lösungsmittel im Vakuum abgezogen. Es entsteht 170 mg von 3-(4-Acetylpiperazin-1-ylmethyl)-7-hydroxy-chromen-4-on (Im) als leicht oranger Feststoff.
1H NMR (300 MHz, DMSO-d6) δ 10.77 (bs, 1H), 8.15 (s, 1H), 7.90 (d, *J*= 8.8 Hz, 1H), 6.92 (dd, *J*= 8.8, 2.3 Hz, 1H), 6.83 (d, *J* = 2.2 Hz, 1H), 3.41 (m, 4H), 3.35 (m, 4H), 2.42 (m, 2H), 2.35 (m, 2H), 1.97 (s, 3H).

## Patentansprüche

1. Nicht-therapeutische Verwendung von Chromon-Derivaten der allgemeinen Formel I oder deren physiologisch unbedenklichen Salze 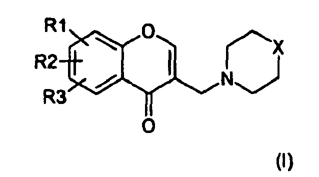 oder einer Zubereitung enthaltend mindestens eine Verbindung der Formel I,
wobei
R₁, R₂ und R₃ -H, -OH, -OA, -A, -OCOA
R₁ und R₂ zusammen können -methylendioxy oder -ethylendioxy sein, R₁, R₂ und R₃ sind identisch oder unterschiedlich mit der Bedingung, dass sie nicht alle gemeinsam -H sein können,
X -O, -CH -R₄, -N-R₄
R₄ -H, -A, -COOH, -COOA, -COA, -CONH₂, -CONHA
A Alkyl, Benzyl, Phenyl
zur Prophylaxe gegen Pigmentstörungen wie Hyperpigmentierung, Sommersprossen, Altersflecken, Sonnenflecken sowie umweltbedingter Hautalterung.

2. Verwendung mindestens eines Chromon-Derivates der Formel I nach Anspruch 1, **dadurch gekennzeichnet, dass** R₁ und R₂ für H und/oder OH stehen.

3. Verwendung mindestens eines Chromon-Derivates der Formel I nach Anspruch 1 und/oder 2, **dadurch gekennzeichnet, dass** R₃ für H oder OH steht.

4. Verwendung nach einem oder mehreren der Ansprüche 1 bis 3, **dadurch gekennzeichnet, dass** es sich bei dem Chromon-Derivat nach Formel I um eine Verbindung ausgewählt aus den Verbindungen mit den Formeln la bis Im handelt:
| | |
|---|---|
| 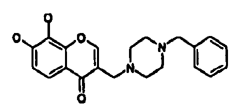 | Ia |
| 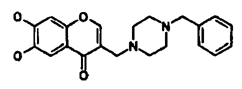 | Ib |
| 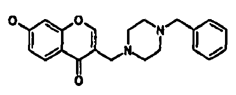 | Ic |
| 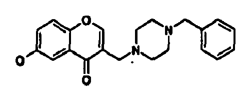 | Id |
| 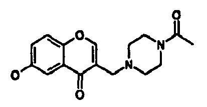 | Ie |
| 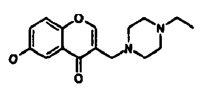 | If |
| 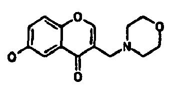 | Ig |
| 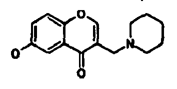 | Ih |
| 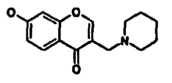 | Ii |
| 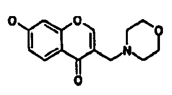 | Ij |
| 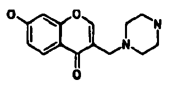 | Ik |
| 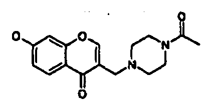 | Im |

5. Zubereitung enthaltend mindestens ein Chromon-Derivat nach Formel I gemäß Anspruch 1, sowie mindestens einen weiteren hautpflegenden Inhaltsstoff und mindestens einen für topische Anwendungen geeigneten Träger.

6. Zubereitung nach Anspruch 5 enthaltend zumindest ein Chromon-Derivat der Formel I, **dadurch gekennzeichnet, dass** die Zubereitungen eine oder mehrere Verbindungen der Formel I in einer Menge von 0.01 bis 20 Gew.%, vorzugsweise in einer Menge von 0.1 bis 10 Gew.%, bezogen auf das Gesamtgewicht der Zubereitung, enthält.

7. Zubereitung nach Anspruch 5 und/oder 6, **dadurch gekennzeichnet, dass** es sich bei mindestens einem weiteren hautpflegenden Inhaltsstoff um eine oder mehrere Antioxidantien und/oder Vitamine handelt.

8. Zubereitung nach einem oder mehreren der Ansprüche 5 bis 7, wobei die Zubereitung einen oder mehrere UV-Filter enthält, die vorzugsweise ausgewählt sind aus der Gruppe, die 3-(4'-Methyl-benzyliden)-dl-kampfer, 1-(4-tert-Butylphenyl)-3-(4-methoxyphenyl)propan-1,3-dion, 4-isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

9. Chromon-Derivate der Formeln Ia bis Im:
| | |
|---|---|
| 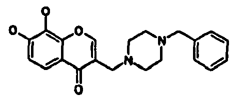 | Ia |
| 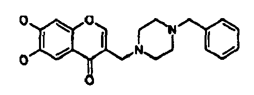 | Ib |
| 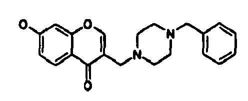 | Ic |
| 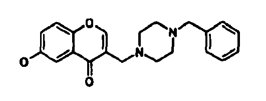 | Id |
| 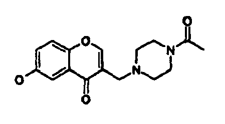 | Ie |
| 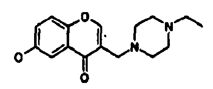 | If |
| 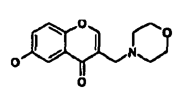 | Ig |
| 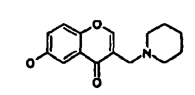 | Ih |
| 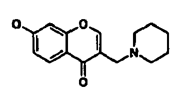 | Ii |
| 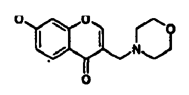 | Ij |
| 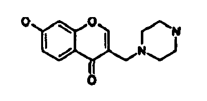 | Ik |
| 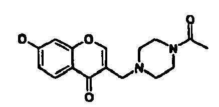 | Im |

## Claims

1. Non-therapeutic use of chromone derivatives of the general formula I or physiologically acceptable salts thereof 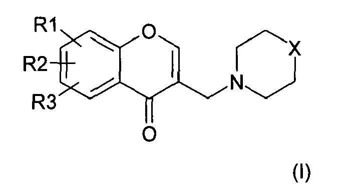 or of a preparation comprising at least one compound of the formula I, where
R₁, R₂ and R₃ are -H, -OH, -OA, -A, -OCOA,
R₁ and R₂ together can be methylenedioxy or ethylenedioxy,
R₁, R₂ and R₃ are identical or different, with the proviso that they cannot all together be -H,
X is -O, -CH-R₄, -N-R₄,
R₄ is -H, -A, -COOH, -COOA, -COA, -CONH₂, -CONHA,
A is alkyl, benzyl, phenyl,
for prophylaxis against pigment defects, such as hyperpigmentation, freckles, age spots, sun spots and environmentally induced skin ageing.

2. Use of at least one chromone derivative of the formula I according to Claim 1, **characterised in that** R₁ and R₂ stand for H and/or OH.

3. Use of at least one chromone derivative of the formula I according to Claim 1 and/or 2, **characterised in that** R₃ stands for H or OH.

4. Use according to one or more of Claims 1 to 3, **characterised in that** the chromone derivative of the formula I is a compound selected from the compounds of the formulae Ia to Im:
| | |
|---|---|
| 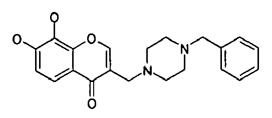 | Ia |
| 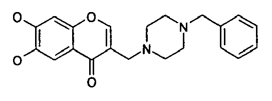 | Ib |
| 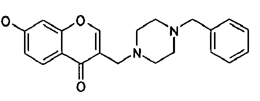 | Ic |
| 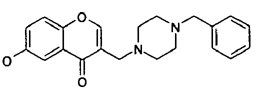 | Id |
| 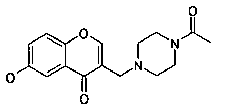 | Ie |
| 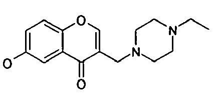 | If |
| 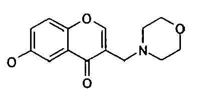 | Ig |
| 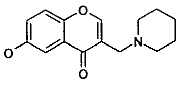 | Ih |
| 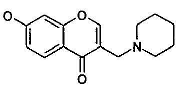 | Ii |
| 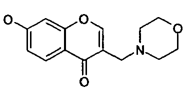 | Ij |
| 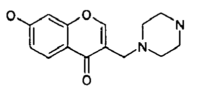 | Ik |
| 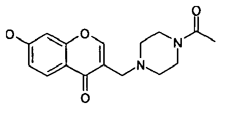 | Im |

5. Preparation comprising at least one chromone derivative of the formula I according to Claim 1 and at least one further skin-care ingredient and at least one vehicle which is suitable for topical applications.

6. Preparation according to Claim 5 comprising at least one chromone derivative of the formula I, **characterised in that** the preparation comprises one or more compounds of the formula I in an amount of 0.01 to 20% by weight, preferably in an amount of 0.1 to 10% by weight, based on the total weight of the preparation.

7. Preparation according to Claim 5 and/or 6, **characterised in that** at least one further skin-care ingredient is one or more antioxidants and/or vitamins.

8. Preparation according to one or more of Claims 5 to 7, where the preparation comprises one or more UV filters, which are preferably selected from the group comprising 3-(4'-methylbenzylidene)-dl-camphor, 1-(4-tert-butylphenyl)-3-(4-methoxyphenyl)propane-1,3-dione, 4-isopropyl-dibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and potassium, sodium and triethanolamine salts thereof.

9. Chromone derivatives of the formulae la to Im:
| | |
|---|---|
| 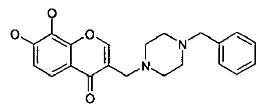 | Ia |
| 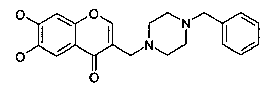 | Ib |
| 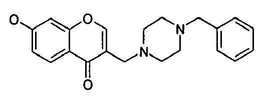 | Ic |
| 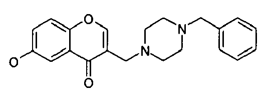 | Id |
| 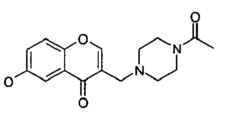 | Ie |
| 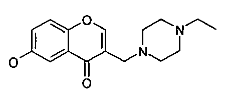 | If |
| 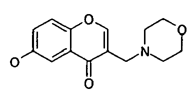 | Ig |
| 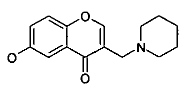 | Ih |
| 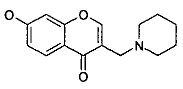 | Ii |
| 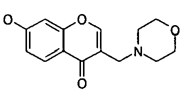 | Ij |
| 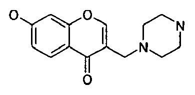 | Ik |
| 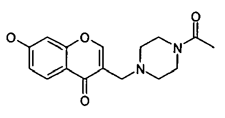 | Im |

## Revendications

1. Utilisation non thérapeutique de dérivés de chromone de formule générale I ou de sels physiologiquement acceptables de ceux-ci 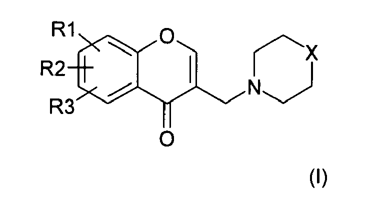 ou d'une préparation comprenant au moins un composé de formule I,
où
R₁, R₂ et R₃ sont -H, -OH, -OA, -A, -OCOA,
R₁ et R₂ peuvent être ensemble méthylènedioxy ou éthylènedioxy,
R₁, R₂ et R₃ sont identiques ou différents, à condition qu'ils ne puissent être ensemble -H,
X est -O, -CH-R₄, -N-R₄,
R₄ est -H, -A, -COOH, -COOA, -COA, -CONH₂, -CONHA,
A est alkyle, benzyle, phényle,
pour une prophylaxie contre les défauts de pigmentation, tels que l'hyperpigmentation, les taches de rousseur, les taches séniles, les taches solaires et le vieillissement cutané induit par l'environnement.

2. Utilisation d'au moins un dérivé de chromone de formule I selon la revendication 1, **caractérisée en ce que** R₁ et R₂ représentent H et/ou OH.

3. Utilisation d'au moins un dérivé de chromone de formule I selon la revendication 1 et/ou 2, **caractérisée en ce que** R₃ représente H ou OH.

4. Utilisation selon l'une ou plusieurs des revendications 1 à 3, **caractérisée en ce que** le dérivé de chromone de formule I est un composé choisi parmi les composés de formules Ia à Im :
| | |
|---|---|
| 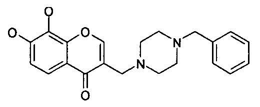 | Ia |
| 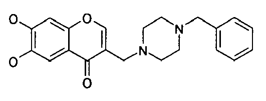 | Ib |
| 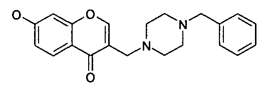 | Ic |
| 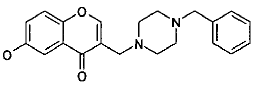 | Id |
| 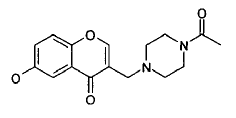 | Ie |
| 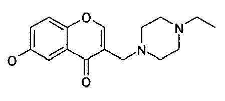 | If |
| 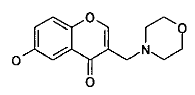 | Ig |
| 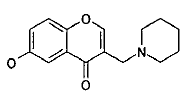 | Ih |
| 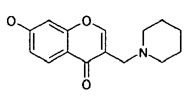 | Ii |
| 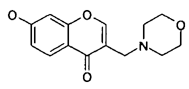 | Ij |
| 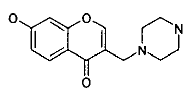 | Ik |
| 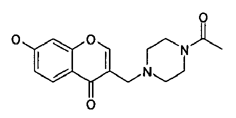 | Im |

5. Préparation comprenant au moins un dérivé de chromone de formule I selon la revendication 1 et au moins un ingrédient de soin de la peau supplémentaire et au moins un véhicule qui est convenable pour des applications topiques.

6. Préparation selon la revendication 5, comprenant au moins un dérivé de chromone de formule I, **caractérisée en ce que** la préparation comprend un ou plusieurs composés de formule I en une quantité allant de 0,01 à 20% en poids, préférablement en une quantité allant de 0,1 à 10% en poids, sur la base du poids total de la préparation.

7. Préparation selon la revendication 5 et/ou 6, **caractérisée en ce que** le au moins un ingrédient de soin de la peau supplémentaire est constitué d'un(e) ou plusieurs antioxydants et/ou vitamines.

8. Préparation selon l'une ou plusieurs des revendications 5 à 7, où la préparation comprend un ou plusieurs filtres UV, qui sont de préférence choisis parmi le groupe constitué par le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, de sodium et de triéthanolamine de ceux-ci.

9. Dérivés de chromone de formules Ia à Im :
| | |
|---|---|
| 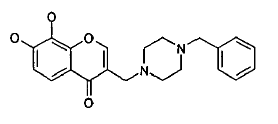 | Ia |
| 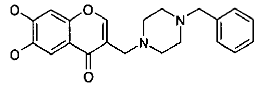 | Ib |
| 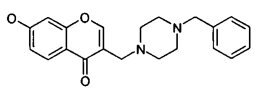 | Ic |
| 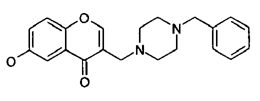 | Id |
| 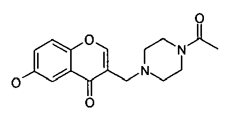 | Ie |
| 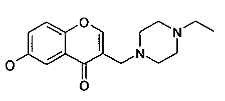 | If |
| 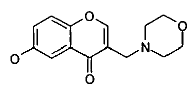 | Ig |
| 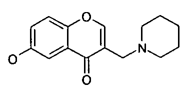 | Ih |
| 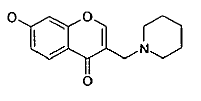 | Ii |
| 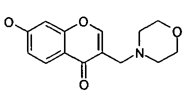 | Ij |
| 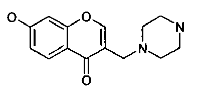 | Ik |
| 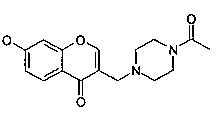 | Im |
